# FASCICULE DE BREVET EUROPEEN

(11) **EP 2 403 413 B1**
(45) Date de publication et mention de la délivrance du brevet: **25.05.2016**
(21) Numéro de dépôt: 10704811.8
(22) Date de dépôt: 16.02.2010
(51) Int. Cl.: A61B 17/02

(54) **DISPOSITIF CHIRURGICAL D'ÉCARTEMENT ET SON PROCÉDÉ DE FABRICATION**
CHIRURGISCHE RETRAKTIONSVORRICHTUNG UND VERFAHREN ZU IHRER HERSTELLUNG
SURGICAL RETRACTING DEVICE AND METHOD FOR THE MANUFACTURE THEREOF

(30) Priorité: 25.02.2009 FR 0951183
(43) Date de publication de la demande: 11.01.2012
(73) Titulaire: Surgical Perspective, 67000 Strasbourg (FR)
(72) Inventeur: RAMOS CLAMOTE, Joachim, F-06000 Nice (FR)
(74) Mandataire: Brizio Delaporte, Allison
(86) Numéro de dépôt international: PCT/EP2010/051906
(87) Numéro de publication internationale: WO 2010/097311

(56) Documents cités:
- EP-A- 1 159 919
- EP-A- 1 413 255
- EP-A- 1 894 529
- US-A1- 2006 122 608
- US-A1- 2008 300 618
- US-B1- 6 506 197

## Description

La présente invention concerne un dispositif chirurgical d'écartement et son procédé de fabrication.

Elle trouvera son application lors d'opérations chirurgicales pour maintenir ou écarter les tissus biologiques et/ou synthétiques, notamment lors d'opérations mini invasives.

Lorsque les chirurgiens réalisent des opérations chirurgicales notamment lors d'une laparoscopie, l'amplitude des mouvements est limitée. Pour atteindre la zone à opérer sans être gênés par les différents organes, les chirurgiens ont recours à divers instruments dont le fil à pois. Cet instrument est composé d'un fil, monté sur une aiguille et muni d'un pois à son autre extrémité. Le pois est formé manuellement par le chirurgien avant ou pendant l'opération à partir de gaze ou de coton. Cette sorte de petite boule de gaze ou de coton est piquée par l'aiguille pour être maintenue par le fil. Ce fil à pois est utilisé en passant l'aiguille à travers un tissu biologique et/ou synthétique jusqu'à ce que le pois vienne au contact du tissu. Puis le chirurgien peut exercer une tension sur le fil pour écarter les tissus.

Un instrument tel que décrit ci-dessus présente l'inconvénient principal d'être fabriqué manuellement de manière artisanale par chaque chirurgien, ce qui leur fait perdre du temps important et ne garantit pas la fiabilité de l'assemblage. Un autre inconvénient est que le chirurgien risque de se piquer durant cette fabrication.

Le document US 2008/300618 montre aussi des dispositifs d'écartement avec les caractéristiques définies dans le préambule de la revendication 1.

Il existe donc le besoin d'un instrument du type fil à pois qui puisse être fabriqué industriellement de manière reproductible.

A cet effet, la présente invention concerne un dispositif chirurgical d'écartement comprenant un élément de liaison tel qu'un fil et un élément d'appui muni d'au moins trois trous débouchants permettant le passage du fil de sorte à solidariser le bouton et le fil par un premier noeud sur une première face de l'élément d'appui et un second noeud sur la seconde face de l'élément d'appui. L'élément d'appui est à titre d'exemple un bouton.

Ce dispositif selon l'invention permet une fabrication industrielle selon un procédé simple. Ainsi, le dispositif chirurgical de fixation est commercialisable sous une forme directement utilisable par le chirurgien.

L'élément d'appui et l'élément de liaison sont solidaires tout en assurant la résistance de l'élément de liaison. En effet, l'élément d'appui ainsi que les noeuds de la première face et de la seconde face sont configurés pour ne pas fragiliser l'élément de liaison.

Selon un mode de réalisation préféré, au moins les trois trous débouchants de l'élément d'appui sont alignés ce qui limite les tensions sur l'élément de liaison et donc les risques de cassure.

D'autres buts et avantages apparaîtront au cours de la description qui suit d'un mode préféré de réalisation de l'invention qui n'en est cependant pas limitatif.

Il convient tout d'abord de rappeler que l'invention concerne un dispositif chirurgical d'écartement comprenant un élément de liaison et un élément d'appui caractérisé par le fait que l'élément de liaison est un fil et que l'élément d'appui comprend une première face, une seconde face et au moins trois trous débouchants, l'élément d'appui et le fil étant solidaires par un premier noeud sur la première face et par un deuxième noeud sur la seconde face.

Selon des variantes préférées de l'invention mais non limitatives, le dispositif est tel que :
- au moins les trois trous débouchants sont alignés,
- l'élément d'appui est circulaire,
- au moins les trois trous sont alignés selon le diamètre de l'élément d'appui,
- l'élément d'appui est de forme allongée,
- au moins les trois trous sont alignés selon l'axe longitudinal de l'élément d'appui,
- l'élément d'appui comprend un trou central et deux trous périphériques,
- le trou central est situé au centre de l'élément d'appui,
- les deux trous périphériques sont équidistants du trou central,
- le premier noeud et le deuxième noeud sont configurés pour maintenir le fil au niveau respectivement de la première face et de la seconde face,
- le premier noeud est constitué par un noeud simple comprenant une portion de fil traversant le premier trou périphérique et une portion de fil traversant le deuxième trou périphérique, une portion de fil entourant une extrémité libre du fil situé du côté de la première face, une portion de fil passant sous la portion de fil entourant l'extrémité libre du fil situé du côté de la première face,
- le deuxième noeud est constitué par une portion de fil traversant le trou central vers la seconde face, une portion de fil entourant la portion de fil formant un pont sur la seconde face.

La présente invention concerne en outre un procédé de fabrication d'un dispositif chirurgical de fixation caractérisé par le fait qu'il comprend les étapes suivantes :
- formation d'un pont sur la première face entre les deux trous périphériques par le passage successif du fil à travers le premier trou périphérique de la première face vers la seconde face puis à travers le second trou périphérique de la seconde face vers la première face,
- formation d'un premier noeud sur la première face,
- passage du fil à travers le trou central de la première face vers la seconde face,
- formation du deuxième noeud sur la seconde face par un tour complet autour du pont de la seconde face.

La figure 1 représente une vue de face de l'élément d'appui selon l'invention.
La figure 2 représente une vue du dispositif chirurgical de fixation selon l'invention.
Les figures 3 à 8 représentent les étapes du procédé de fabrication du dispositif chirurgical selon la figure 2.

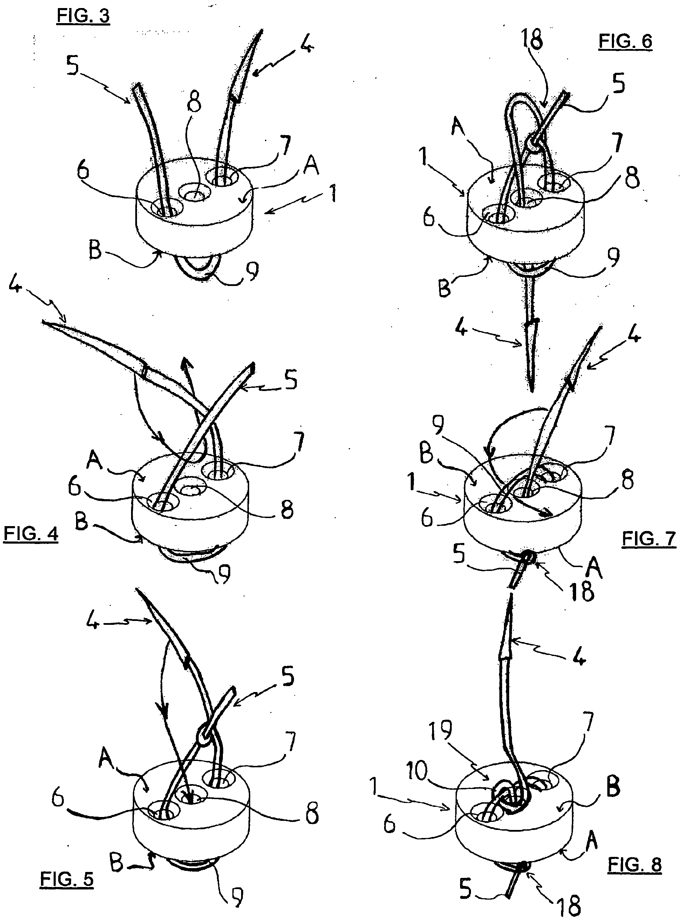

Les figures 3 à 5 et 9 à 11 représentent la formation du premier noeud.
Les figures 6 à 8 et 12 à 14 représentent la formation du deuxième noeud.
   Sur les figures 3 à 6 et 9 à 12, l'élément d'appui est représenté la première face vers le haut. Alors que sur les figures 7, 8 et 13, 14, l'élément d'appui est représenté selon une vue inversée, la première face vers le bas.

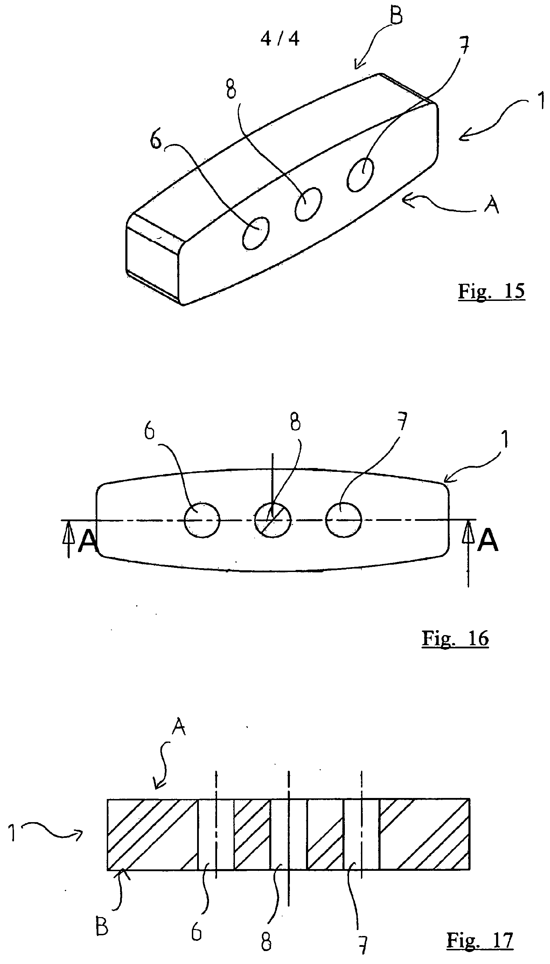

Les figures 15 à 17 représentent un élément d'appui selon un deuxième mode de réalisation de l'invention.
La figure 15 est une vue en perspective.
La figure 16 est une vue de face.
La figure 17 est une vue selon la coupe AA de la figure 16.

La suite de la description utilisera les termes "fil" et "bouton" sans pour autant que cela soit limitatif. Ces termes pouvant être généralisés par "élément de liaison" et "élément d'appui".

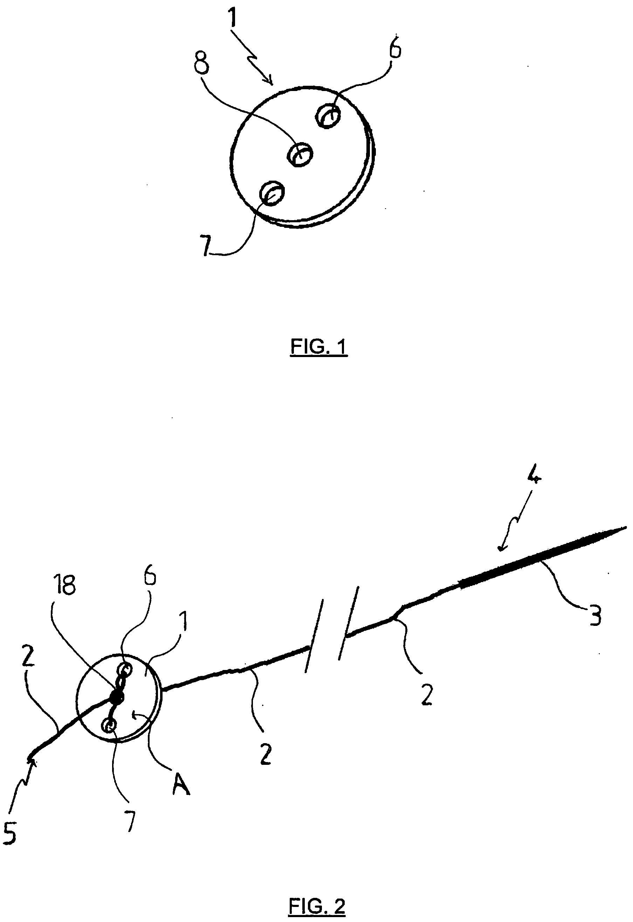

Comme représenté en figure 2, le dispositif chirurgical de fixation comprend deux éléments principaux, un élément de liaison préférentiellement un fil 2 et un élément d'appui préférentiellement un bouton 1.

Le fil 2 est préférentiellement une tresse en polyester téflonisé permettant de limiter les forces de friction.

Le bouton 1 est préférentiellement fabriqué à base de silicone. Le bouton 1 comprend une première face A et une seconde face B.

De manière caractéristique, le bouton 1 comprend au moins trois trous 6, 7, 8 débouchants permettant le passage du fil 2 Selon un autre mode de réalisation, le bouton 1 comprend plus de trois trous. Le fil 2 passe avantageusement au travers des différents trous 6, 7, 8 du bouton 1 de sorte à former un premier noeud 18 sur la première face A du bouton et un deuxième noeud 19 sur la seconde face B du bouton 1. Le fil 2 est ainsi solidaire du bouton 1.

De manière préférentielle, le fil 2 est muni à l'une de ses extrémités d'une aiguille 3. Cette aiguille transperce la paroi abdominale, le fil est ensuite immobilisé à l'extérieur du corps du patient et permet notamment l'écartement des tissus biologiques et/ou synthétiques par le dispositif chirurgical selon l'invention lors des opérations. A l'opposé de l'extrémité munie de l'aiguille 3 (extrémité aiguillée 4), le fil 2 a une extrémité libre 5.

D'autres modes de réalisation peuvent être envisagés. Notamment, au lieu d'utiliser un fil 2 muni d'une aiguille 3, des moyens électromagnétiques positionnés d'une part sur le bouton 1 et d'autre part sur un élément complémentaire, peuvent être prévus. Le bouton 1 et l'élément complémentaire sont disposés de part et d'autre d'un tissu biologique et/ou synthétique à écarter. Le bouton 1 est préférentiellement placé sur la face interne du tissu à écarter permettant au praticien d'exercer une tension sur le fil de sorte à écarter le tissu. Il peut aussi être prévu des moyens d'accrochage tels que des pinces miniatures.

De manière avantageuse, les trois trous 6, 7, 8 sont alignés.

Le bouton 1 peut être circulaire tel que représenté sur l'ensemble des figures. Dans ce cas, les trois trous 6, 7, 8 sont alignés préférentiellement selon le diamètre du bouton 1.

Selon une autre possibilité non représentée, le bouton 1 est de forme allongée du type ovoïde permettant une meilleure utilisation lors d'opérations avec des trocarts de faible diamètre. Selon cette possibilité, les trois trous 6, 7, 8 sont alignés préférentiellement selon l'axe longitudinal du bouton 1.

Cet alignement des trois trous 6, 7, 8 permet la réalisation avantageuse du premier noeud 18 et du deuxième noeud 19 sensiblement au centre du bouton 1 conduisant ainsi à un centrage du fil 2 notamment de son extrémité aiguillée 4 qui permettra une bonne application de l'élément d'appui du bouton 1, sur le tissu biologique et/ou synthétique à écarter.

Les trois trous 6, 7, 8 alignés sont préférentiellement constitués par un trou central 8 et deux trous périphériques 6, 7. Avantageusement, le trou central 8 est situé au centre du bouton 1. On entend par "centre" le centre du cercle si le bouton 1 est circulaire ou le centre de gravité du bouton 1 si celui-ci a une forme différente.

Selon un mode de réalisation avantageux, les trois trous 6, 7, 8 sont répartis de manière symétriques sur le diamètre du bouton 1 ou sur son axe longitudinal.

Le déposant s'est aperçu que le mode de réalisation dans lequel les deux trous périphériques 6, 7 sont équidistants du trou central 8 permet un bon équilibre du bouton 1 lors de son utilisation comme élément d'appui. En effet, cette disposition permet un positionnement équilibré du premier noeud 18 et du deuxième noeud 19.

Le premier noeud 18 est situé sur la première face A du bouton 1, il est réalisé en premier lors du procédé de fabrication du dispositif chirurgical selon l'invention. Le premier noeud 18 est préférentiellement du type noeud simple. Il est configuré pour être centré relativement au bouton 1; c'est-à-dire selon certains modes de réalisation, qu'il est positionné sensiblement au niveau du trou central 8.

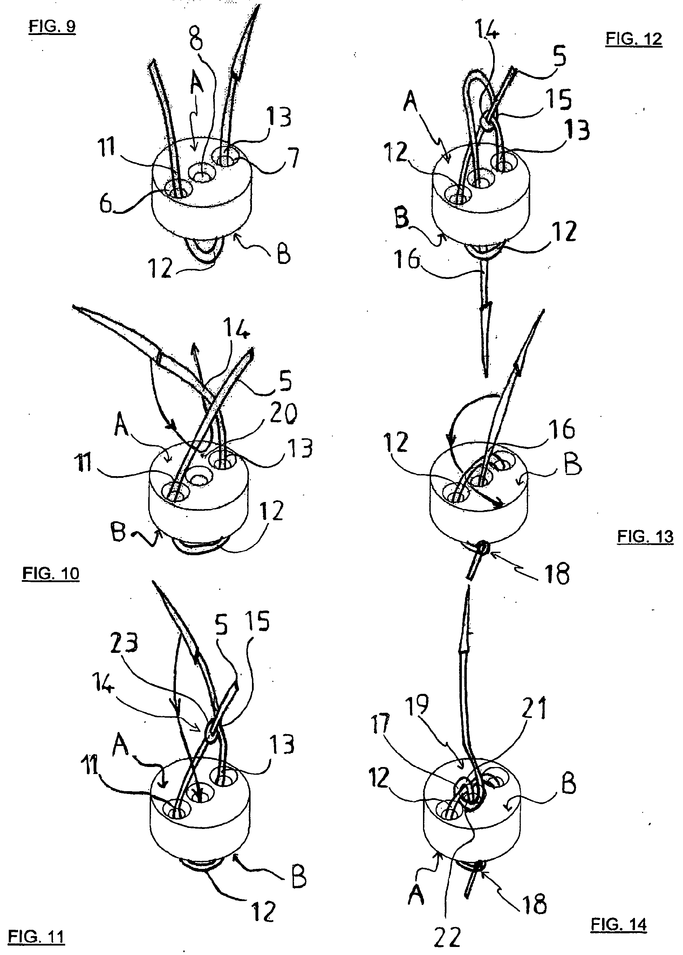

Les figures 9, 10 et 11 représentent les étapes de fabrication du premier noeud 18. Tout d'abord, le fil 2 passe au travers des trous périphériques 6, 7 et forme un pont 9 sur la seconde face du bouton 1. Pour cela, l'extrémité aiguillée 4 du fil 2 est préférentiellement introduite au travers du premier trou périphérique 6 de la première face A vers la seconde face B. Puis l'extrémité aiguillée 4 du fil 2 est introduite au travers du deuxième trou périphérique 7 de la seconde face B vers la première face A.

Les deux extrémités 4 et 5 du fil 2 se retrouvent au niveau de la première face A du bouton 1. Ces deux extrémités vont être associées de sorte à réaliser un noeud simple. Préférentiellement, l'extrémité aiguillée 4 entoure l'extrémité libre 5 du fil 2 de sorte à former une boucle 20 au travers de laquelle l'extrémité aiguillée 4 est introduite pour ressortir de la boucle 20. Le premier noeud 18 est serré en tirant de manière équilibrée sur les deux extrémités 4, 5 du fil 2. Préférentiellement, le premier noeud 18 se positionne au niveau du trou central 8. Ce premier noeud a un rôle de solidarisation du fil 2 au bouton 1. Il permet en outre de centrer le fil 2 au niveau du trou central 8 pour permettre la formation du second noeud au niveau de la seconde face B du bouton 1. La disposition du premier noeud 18 traversant les deux trous périphériques 6, 7 permet en outre de répartir les forces de traction sur l'ensemble de la surface du bouton 1.

Comme représenté en figure 6, une fois le premier noeud 18 réalisé sur la première face A du bouton 1, l'extrémité aiguillée 4 du fil 2 est introduite au travers du trou central 8 de la première face A vers la seconde face B. Au niveau de la seconde face B, l'extrémité aiguillée 4 va passer successivement par-dessus le pont 9 puis en dessous du pont 9 pour former un tour complet à 360° autour du pont 9. Ce deuxième noeud 19 permet un blocage du fil 2 sur le bouton 1. En effet, lors de la fabrication puis de son utilisation, une tension est exercée sur le fil 2 par son extrémité aiguillée 4 resserrant le premier noeud et rapprochant le pont 9 de la surface de la seconde face B du bouton 1 de sorte à appuyer et à bloquer la portion de fil 22 située sous le pont 9.

Le deuxième noeud 19 a aussi un rôle de centrage du fil 2. En effet, le fil 2 est issu du trou central 8 de la seconde face B dans une position centrale, le deuxième noeud 19 permet de maintenir le fil 2 dans cette position centrale. Cette position centrale du fil 2 relativement au bouton 1 permet de positionner correctement le bouton 1 lors de son utilisation sur les tissus biologiques et/ou les tissus synthétiques.

D'un point de vue structurel, le premier noeud est constitué d'un noeud simple et comprend : une portion de fil 11 traversant le premier trou périphérique 6 de la première face A vers la seconde face B, une portion de fil 12 formant un pont 9 sur la seconde face B entre le premier trou périphérique 6 et le deuxième trou périphérique 7, une portion de fil 13 traversant le second trou périphérique 7 de la seconde face B vers la première face A, une portion de fil 14 entourant l'extrémité libre 5 du fil 2. Cette portion de fil 14 se distingue en une portion de fil 23 passant par dessus l'extrémité libre 5 du fil 2 formant une boucle 20 dans laquelle passe l'extrémité aiguillée 4 : c'est la portion de fil 15 située sous l'extrémité libre 5.

Le deuxième noeud 19 est constitué d'une portion de fil 16 traversant le trou central 8 de la première face A vers la seconde face B et d'une portion de fil 17 entourant la portion de fil 12 constituant le pont 9. Cette portion de fil 17 est constituée d'une portion de fil 21 située sur la portion de fil 12 et une portion de fil 22 située sous la portion de fil 12.

Le dispositif chirurgical de fixation selon l'invention est utilisable notamment lors d'opérations mini invasives quand le chirurgien doit opérer une zone peu accessible et doit maintenir des organes écartés de la zone d'opération. Selon un premier mode d'utilisation, le dispositif permet de maintenir un filet contre le tissu biologique pour l'écarter. Le chirurgien passera ainsi l'aiguille au travers un tissu synthétique tel qu'un filet puis au travers de la paroi abdominale de sorte à écarter le tissu biologique. Le bouton 1 permet de former une zone d'appui au niveau du tissu biologique et/ou synthétique et le fil 2 permet de réaliser la traction pour maintenir le tissu écarté.

Selon une autre possibilité, le chirurgien peut utiliser le dispositif selon l'invention sans élément supplémentaire, pour cela il traversera grâce à l'aiguille 3 un tissu biologique contre lequel vient s'appuyer le bouton 1 lui permettant ainsi d'exercer une traction sur le tissu biologique à écarter grâce au fil 2.

Le dispositif selon l'invention, notamment le premier noeud 18 et le deuxième noeud 19, permet que le fil 2 et le bouton 1 soient solidaires même si le fil 2 se casse au niveau d'une de ses extrémités ce qui permet au chirurgien de récupérer l'ensemble bouton 1 - fil 2 de manière simultanée sans risquer de perdre les éléments du dispositif dans le corps du patient.

Le dispositif selon l'invention peut être radio opaque ou non, il est peut être en matériau résorbable ou non.

A titre d'exemple, un bouton 1 de type circulaire peut avoir un diamètre d'environ 9 millimètres pour une épaisseur d'environ 3 millimètres. Si le bouton est de forme allongée, la longueur du bouton 1 est de l'ordre de 15 millimètres.

Préférentiellement, les trous ont un diamètre d'environ 1,5 millimètres. Selon un mode de réalisation où les trous 6, 7, 8 sont alignés et équidistants, l'espace entre chaque trou est de l'ordre de 1,13 millimètre. La distance est préférentiellement la même entre les trous 6 et 7 et le pourtour du bouton 1. Préférentiellement, le fil 2 a une longueur comprise entre 9 et 10 centimètres entre le deuxième noeud 19 et son extrémité aiguillée. L'aiguille 3 est préférentiellement d'une taille de 50 millimètres.

### REFERENCES

- 1.: Elément d'appui / Bouton
- 2.: Fil
- 3.: Aiguille
- 4.: Extrémité aiguillée
- 5.: Extrémité libre
- 6.: 1^{er} trou périphérique
- 7.: 2^{éme} trou périphérique
- 8.: Trou central
- 9.: Pont
- 10.: Tour
- 11.: Portion de fil traversant le 1^{er} trou périphérique
- 12.: Portion de fil formant un pont sur la seconde face entre le 1^{er} et le 2^{ème} trou périphérique
- 13.: Portion de fil traversant le 2^{ème} trou périphérique
- 14.: Portion de fil entourant l'extrémité libre du fil
- 15.: Portion de fil passant sous la portion de fil 14
- 16.: Portion de fil traversant le trou central vers la seconde face
- 17.: Portion de fil entamant la portion de fil 12
- 18.: Premier noeud
- 19.: Deuxième noeud
- 20.: Boucle formée par les portions de fils 11-13 et face A
- 21.: Portion de fil située sur la portion 12
- 22.: Portion de fil située sous la portion 12
- 23.: Portion de fil située sur l'extrémité libre 5
- A.: Première face
- B.: Seconde face

## Revendications

1. Dispositif chirurgical d'écartement comprenant un élément de liaison et un élément d'appui l'élément de liaison étant un fil (2) et l'élément d'appui (1) comprenant une première (A) et une seconde (B) face, l'élément d'appui et le fil étant solidaires par un premier noeud (18) sur la première face (A), **caractérisé par le fait que** l'élément d'appui comprend au moins trois trous (6, 7, 8) débouchants, dans lesquels passe le fil (2), l'élément d'appui (1) et le fil (2) étant solidaires par un deuxième noeud (19) sur la seconde face (B).

2. Dispositif selon la revendication 1 dans lequel au moins les trois trous (6, 7, 8) débouchants sont alignés.

3. Dispositif selon l'une des revendications 1 ou 2 dans lequel l'élément d'appui (1) est circulaire.

4. Dispositif selon la revendication 2 en combinaison avec la revendication 3 dans lequel au moins les trois trous (6, 7, 8) sont alignés selon un diamètre de l'élément d'appui (1).

5. Dispositif selon l'une quelconque des revendications 1 ou 2 dans lequel l'élément d'appui (1) est de forme allongée.

6. Dispositif selon la revendication 2 en combinaison avec la revendication 5 dans lequel au moins les trois trous (6, 7, 8) sont alignés selon l'axe longitudinal de l'élément d'appui (1).

7. Dispositif selon l'une quelconque des revendications précédentes dans lequel l'élément d'appui (1) comprend un trou central (8) et deux trous périphériques (6, 7).

8. Dispositif selon la revendication 7 dans lequel le trou central est situé au centre de l'élément d'appui (1).

9. Dispositif selon l'une quelconque des revendications 7 ou 8 dans lequel les deux trous périphériques (6, 7) sont équidistants du trou central (8).

10. Dispositif selon une quelconque des revendications précédentes dans lequel le premier noeud (18) et le deuxième noeud (19) sont configurés pour maintenir le fil (2) au niveau respectivement de la première face (A) et de la seconde face (B).

11. Dispositif selon l'une quelconque des revendications 7 à 10 dans lequel le premier noeud (18) est constitué par un noeud simple comprenant une portion de fil (11) traversant le premier trou périphérique (6) et une portion de fil (13) traversant le deuxième trou périphérique (7), une portion de fil (14) entourant une extrémité libre (5) du fil (2) situé du côté de la première face (A), une portion de fil passant sous la portion de fil entourant l'extrémité libre (5) du fil (2) situé du côté de la première face (A).

12. Dispositif selon la revendication 11 dans lequel le deuxième noeud (19) est constitué par une portion de fil traversant le trou central (8) vers la seconde face, une portion de fil entourant la portion de fil formant un pont (9) sur la seconde face.

13. Procédé de fabrication d'un dispositif chirurgical de fixation selon la revendication 12 **caractérisé par le fait qu'**il comprend les étapes suivantes :
- formation d'un pont (9) sur la première face entre les deux trous périphériques (6, 7) par le passage successif du fil à travers le premier trou périphérique (6) de la première face (A) vers la seconde face (B) puis à travers le second trou périphérique (7) de la seconde face (B) vers la première face (A),
- formation d'un premier noeud (18) sur la première face (A),
- passage du fil (2) à travers le trou central (8) de la première face (A) vers la seconde face (B),
- formation du deuxième noeud (19) sur la seconde face (B) par un tour complet autour du pont (9) de la seconde face (B).

## Patentansprüche

1. Chirurgische Spreizvorrichtung, die ein Verbindungselement und ein Auflageelement umfasst, wobei das Verbindungselement ein Draht (2) ist und das Auflageelement (1) eine erste (A) und eine zweite (B) Fläche aufweist, wobei das Auflageelement und der Draht durch einen ersten Knoten (18) auf der ersten Fläche (A) miteinander verbunden sind, **dadurch gekennzeichnet, dass** das Auflageelement mindestens drei Löcher (6, 7, 8) aufweist, durch welche der Draht (2) hindurch geführt wird, wobei das Auflageelement (1) und der Draht (2) durch einen zweiten Knoten (19) auf der zweiten Fläche (B) miteinander verbunden sind.

2. Vorrichtung nach Anspruch 1, in der sich die mindestens drei Löcher (6, 7, 8) auf einer Linie befinden.

3. Vorrichtung nach einem der Ansprüche 1 oder 2, in der das Auflageelement (1) rund ist.

4. Vorrichtung nach Anspruch 2 in Verbindung mit Anspruch 3, in der sich die mindesten drei Löcher (6, 7, 8) innerhalb des Durchmessers des Auflageelements (1) auf einer Linie befinden.

5. Vorrichtung nach einem der Ansprüche 1 oder 2, in dem das Auflageelement (1) eine längliche Form hat.

6. Vorrichtung nach Anspruch 2 in Verbindung mit Anspruch 5, in der die mindestens drei Löcher (6, 7, 8) auf einer Längsachse des Auflageelements (1) angeordnet sind.

7. Vorrichtung nach einem der vorhergehenden Ansprüche, in der das Auflageelement (1) ein mittleres Loch (8) und zwei äußere Löcher (6, 7) aufweist.

8. Vorrichtung nach Anspruch 7, in welcher sich das mittlere Loche in der Mitte des Auflageelements (1) befindet.

9. Vorrichtung nach einem der Ansprüche 7 oder 8, in dem die beiden äußeren Löcher (6, 7) abstandsgleich vom mittleren Loch (8) gelegen sind.

10. Vorrichtung nach einem der vorhergehenden Ansprüche, in welcher der erste Knoten (18) und der zweite Knoten (19) so angeordnet sind, dass sie den Draht (2) jeweils in Höhe der ersten Fläche (A) und der zweiten Fläche (B) halten.

11. Vorrichtung nach einem der Ansprüche 7 bis 10, in welcher der erste Knoten (18) aus einem einfachen Knoten besteht, welcher einen Drahtabschnitt (11) aufweist, der durch das erste äußere Loch (6) geführt wird, einen Drahtabschnitt (13), der durch das zweite äußere Loch (7) geführt wird, einen Drahtabschnitt (14), der um ein freies Ende (5) des Drahtes (2) auf der Seite der ersten Fläche (A) gewickelt ist, und einen Drahtabschnitt, welcher unterhalb des Drahtabschnitts verläuft, der um das freie Ende (5) von Draht (2) auf der Seite der ersten Fläche (A) gewickelt ist.

12. Vorrichtung nach Anspruch 11, in welcher der zweite Knoten (19) aus einem Drahtabschnitt besteht, der durch das mittlere Loch (8) zur zweiten Fläche führt, und einen Drahtabschnitt, der um den Drahtabschnitt gewickelt ist, welcher eine Brücke (9) auf der zweiten Fläche bildet.

13. Verfahren für die Herstellung einer chirurgischen Befestigungsvorrichtung nach Anspruch 12, **dadurch gekennzeichnet, dass** es folgende Schritte umfasst:
- die Bildung einer Brücke (9) auf der ersten Fläche zwischen den beiden äußeren Löchern (6, 7), indem der Draht durch das erste äußere Loch (6) der ersten Fläche (A) zur zweiten Fläche (B) und dann durch das zweite äußere Loch (7) der zweiten Fläche (B) zur ersten Fläche (A) geführt wird,
- die Bildung eines ersten Knotens (18) auf der ersten Fläche (A),
- das Durchführen des Drahts (2) durch die mittlere Öffnung (8) der ersten Fläche (A) zur zweiten Fläche (B).
- die Bildung des zweiten Knotens (19) auf der zweiten Fläche (B) durch eine ganze Umwicklung der Brücke (9) der zweiten Fläche (B).

## Claims

1. A surgical spreading device comprising a linking element and a support element, with the linking element being a thread (2) and the support element (1) comprising a first (A) and a second (B) face, with the support element and the thread being interlinked by means of a first knot (18) on the first face (A), **characterized in that** the support element comprises at least three through holes (6, 7, 8) wherethrough the thread (2) passes, with the support element (1) and the thread (2) being interlinked by means of a second knot (19) on the second face (B).

2. A device according to claim 1, wherein at least the three through holes (6, 7, 8) are aligned.

3. A device according to one of claims 1 or 2, wherein the support element (1) is circular.

4. A device according to claim 2 in combination with claim 3, wherein at least the three through holes (6, 7, 8) are aligned along a diameter of the support element (1).

5. A device according to any one of claims 1 or 2, wherein the support element (1) has an elongated shape.

6. A device according to claim 2 in combination with claim 5, wherein at least the three through holes (6, 7, 8) are aligned along the longitudinal axis of the support element (1).

7. A device according to any one of the preceding claims, wherein the support element (1) comprises a central hole (8) and two peripheral holes (6, 7).

8. A device according to claim 7, wherein the central hole is located at the center of the support element (1).

9. A device according to any one of claims 7 or 8, wherein the two peripheral holes (6, 7) are equidistant from the central hole (8).

10. A device according to any one of the preceding claims, wherein the first knot (18) and the second knot (19) are so configured as to hold the thread (2) at the level of the first face (A) and of the second face (B), respectively.

11. A device according to any one of claims 7 to 10, wherein the first knot (18) consists of a single knot comprising a portion of thread (11) passing through the first peripheral hole (6) and a portion of thread (13) passing through the second peripheral hole (7), with a portion of the thread (14) surrounding a free end (5) of the thread (2) located on the first face (A) side, with a portion of thread passing under the portion of thread surrounding the free end (5) of the thread (2) located on the first face (A) side.

12. A device according to claim 11, wherein the second knot (19) consists of a portion of thread passing through the central hole (8) toward the second face, with a portion of thread surrounding the portion of thread forming a bridge (9) on the second face.

13. A method for manufacturing a surgical fastening device according to claim 12, **characterized in that** it comprises the following steps:
- forming a bridge (9) on the first face between the two peripheral holes (6, 7) by means of the successive passage of the thread through the first peripheral hole (6) of the first face (A) towards the second face (B) and then through the second peripheral hole (7) of the second face (B) towards the first face (A),
- forming a first knot (18) on the first face (A),
- passing the thread (2) through the central hole (8) of the first face (A) towards the second face (B),
- forming the second knot (19) on the second face (B) by means of one full turn around the bridge (9) of the second face (B).
